(19) Europäisches Patentamt
European Patent Office
Office européen des brevets

(11) **EP 3 427 764 A1**

(12) **EUROPEAN PATENT APPLICATION**

(43) Date of publication:
**16.01.2019 Bulletin 2019/03**

(51) Int Cl.:
*A61L 27/22* (2006.01)          *A61L 27/34* (2006.01)
*A61L 27/50* (2006.01)          *A61L 27/54* (2006.01)
*A61L 27/56* (2006.01)

(21) Application number: **17305917.1**

(22) Date of filing: **12.07.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
• **Université de Technologie de Compiègne**
  **60203 Compiègne Cedex (FR)**
• **CENTRE NATIONAL DE LA RECHERCHE SCIENTIFIQUE**
  **75016 Paris (FR)**
• **Université de Strasbourg**
  **67000 Strasbourg (FR)**
• **Université de Picardie Jules Verne**
  **80025 Amiens Cedex 1 (FR)**

• **Centre Hospitalier Universitaire D'Amiens - Picardie**
  **80000 Amiens (FR)**

(72) Inventors:
• **DEVAUCHELLE, Bernard**
  **80000 AMIENS (FR)**
• **HEBRAUD, Anne**
  **67280 OBERHASLACH (FR)**
• **SCHLATTER, Guy**
  **67460 SOUFFELWEYERSHEIM (FR)**
• **BELANGER, Kayla**
  **HUDSON, NH 03051 (US)**
• **EGLES, Christophe**
  **60200 COMPIEGNE (FR)**

(74) Representative: **Gevers & Orès**
  **41 avenue de Friedland**
  **75008 Paris (FR)**

(54) **FIBROUS POLYMER MATERIAL COMPRISING FIBROIN AND POLYMER SCAFFOLDS COMPRISING THEREOF**

(57)     The invention pertains to the field of biomaterials and their use, in particular for the production of implants to support the growth and recovery of biological tissues. The invention more particularly pertains to a fibrous polymer material comprising a first layer of aligned fibroin fiber, a second layer of fibroin fibers, wherein the fibroin fibers are randomly oriented within said second layer, a third layer of aligned fibroin fibers; and polymer scaffolds comprising thereof.

FIGURE 2

## Description

The invention pertains to the field of biomaterials and their use, in particular for the production of implants to support the growth and recovery of biological tissues. The invention more particularly pertains to a fibrous polymer material comprising fibroin fibers, and polymer scaffolds comprising thereof.

## PRIOR ART

The nervous system is made up of the central nervous system (CNS) and the peripheral nervous system (PNS). The CNS includes the brain and spinal cord while the PNS includes all nerves that branch out from the CNS. Nerves in the PNS contain sensory, or afferent, neurons that carry information to the CNS and motor, or efferent, neurons that transmit information from the CNS. While the cell bodies of motor and sensory neurons are located in the spinal cord and in dorsal root ganglia respectively, these neurons have long extensions called axons that innervate the various organs throughout the body. Due to environmental constraints in the CNS, spontaneous neuronal repair after injury does not occur. However, the PNS differs in this characteristic supporting axon regeneration after trauma with or without surgical intervention.

After a severe injury to a peripheral nerve, the axon segments distal to the trauma begin to degrade and one may experience loss of function locally as muscles as well as the skin are denervated during a process called Wallerian degeneration. Once the distal axon segments degrade, the proximal axon stubs begin to regrow toward their respective innervation targets assisted by biochemical cues and structural guidance from proliferating Schwann cells. In many minor nerve injury cases, nerves will regenerate naturally resulting in functional recovery without the need of surgical intervention. However, for more serious injuries a nerve graft must be implanted in order to bridge the gap between the proximal and distal segments of the nerve.

The gold standard procedure for treatment after a severe nerve injury is the nerve autograft, which consists of grafting the damaged nerve with a nerve sample taken directly from the patient. However, this technique has several drawbacks such as the need for multiple operations, limited availability, loss of function at the donor site, and the risk of developing a painful neuroma at the donor site.

To overcome these issues, synthetic nerve guidance conduits (NGCs) have been developed to replace the autograft, but no system has been able to consistently demonstrate adequate superiority.

Tensile strength of the NGC is a very important parameter to consider since excessive tension on an implant after surgery can lead to poor regeneration. Therefore, the implant must uphold a certain stability while under stress in order to achieve a conduit with a relatively small length and an environment that will promote healthy neuron growth.

The natural polymer fibroin has shown to be a promising material for the fabrication of NGCs since it is biocompatible, biodegradable, easily functionalized, easily chemically modified, and has robust mechanical properties compared to other natural materials.

However, the manufacturing of fibroin NGCs which present with properties similar to that of autografts has not yet been reported, likely because of the numerous technical difficulties to overcome in this process.

There is thus a need for an improved biomaterial based on fibroin, which may be used to manufacture improved NGCs.

## DESCRIPTION

The inventors have set up a fibrous polymer material comprising fibroin fibers, which possesses a specific structure making it particularly useful when used as an implant. The fibrous polymer material of the invention is composed of several layers of fibroin polymer, wherein the fibers are arranged in such a way as to promote tissue recovery, while retaining great tensile strength. This effect is obtained by a three layer structure wherein the two outer layers comprise aligned fibroin fibers, and the internal layer comprises randomly arranged fibroin fibers.

The alignment of the fibroin fibers of the outer layers promote optimal tissue recovery by directing cellular growth. The random arrangement of the fibroin fibers provides appropriate tensile strength to the fibrous polymer material, thus further enhancing tissue regeneration. The fibrous polymer material of the invention is particularly suited for the preparation of polymer scaffolds which may be used as implants, in particular as nerve guidance conduits (NGCs).

The polymer scaffolds prepared with the fibrous polymer material of the invention present with exceptional properties, which make them particularly suited for use as NGC. In particular, the polymer scaffolds of the invention present with very good resistance to elongation, comparable to the native nerve. In addition, they show interesting elasticity before permanent deformation, and are less brittle than polymer scaffolds prepared with less complex fibroin material.

In a first embodiment, the invention pertains to a fibrous polymer material, comprising

- a first layer of aligned fibroin fibers;
- a second layer of fibroin fibers, wherein the fibroin fibers are randomly oriented within said second layer ;
- a third layer of aligned fibroin fibers.

In the context of the invention, a " layer of aligned fibroin fibers " refers to a layer comprising fibroin fibers, wherein the majority of the fibroin fibers, that is to say more than 90%, preferably more than 95% of the fibroin fibers, are parallel to each other within said layer. The term parallel should not be construed strictly, and it should be understood that two fibroin fibers may still be considered parallel when their respective directions form an angle of less than 10 arc degrees.

In the context of the invention, it should be understood that the first, second and third layers are organized within the fibrous polymer material in this order.

In the context of the invention, fibroin may for example be obtained from the silk produced by *Lepidopteran larvae* such as silkworms, such as for instance silkworms form the species *Bombyx mori, Anthéroea Yama-Maî* and *Anthéroea Pernyï;* moth, such as moth from the genera *Antheraea, Cricula, Samia* and *Gonometa,* spiders, ants or bees. Preferably, the fibroin used in the invention is from silkworms, preferably silkworms chosen form a species chosen in the list consisting of *Bombyx mori, Anthéroea Yama-Maî* and *Anthéroea Pernyï,* yet preferably from *Bombyx mori.*

Silk in its raw state consists of two main proteins, sericin and fibroin, and fibroin may be obtained by treatment of the silk to remove sericin. Typically, fibroin is purified from silk using a simple alkali or enzyme based degumming procedure (Kundu et al., Advanced Drug Delivery Reviews, 65(4): 457-470, 2013).

Layers of aligned fibers and randomly arranged fibers may be obtained by electrospinning, using methods known in the art, such as for instance thoroughly detailed in Wittmer et al. (Acta Biomater.;7(10):3789-95; 2011).

Advantageously, the fibroin fibers have a diameter of between 100 nm and 900 nm, preferably of between 250 nm and 650 nm.

Preferably, the fibrous polymer material of the invention has the shape of a sheet. Advantageously, the fibrous polymer material of the invention has a thickness of between 0.05 and 0.15 mm, yet preferably of between 0.07 and 0.1 mm. Preferably, in the material of the invention, all three layers have the same thickness.

It has been shown that functionalized silk fibers can help promote axon regeneration in the central nervous system. In an advantageous embodiment, the fibrous polymer material is functionalized by the addition of at least one biological molecule, preferably chosen from the list consisting of nerve growth factor (NGF), ciliary neurotrophic factor (CNTF), epidermal growth factor (EGF), neurotrophin 3 (NT-3), brain derived neurotrophic factor (BDNF) and Neurotrophin 4/5 (NT-4/5). The preparation of such functionalized fibrous polymer material may be performed using methods known in the art, such as for instance disclosed in Wittmer et al. (Adv Funct Mater.; 21(22):4202; 2011) or Dinis et al. (PLoS One.; 9(10):e109770, 2014).

Concentration gradients of biological molecules influence cell behavior and growth in biological tissues. Preferably, when the fibrous polymer material is functionalized, the at least one biological molecule is present as a concentration gradient, yet preferably as an increasing concentration gradient, along the aligned fibers of the first and the third layers of the material. Such gradient may easily be obtained using methods known in the art, in particular those disclosed in Dinis et al. (ACS Appl Mater Interfaces; 6(19):16817-26. 2014).

The inventors have further defined the optimal conditions to prepare the fibrous polymer material of the invention. Said optimal conditions comprise notably the preparation of a solution of fibroin with the appropriate concentration, further comprising poly(ethylene oxide), followed by the electrospinning of this solution according to a specific scheme, in conditions appropriate for the formation of a fibrous polymer material according to the invention.

In a second embodiment, the invention pertains to a method for producing a fibrous polymer material according to the invention, comprising preparing a solution of fibroin and poly(ethylene oxide), and forming fibroin fibers by electrospinning said solution.

Preferably, the method disclosed above comprises:

- preparing a solution of fibroin, wherein the solution comprises between 4% and 15% fibroin, and further comprises poly(ethylene oxide), and ;
- forming fibroin fibers by electrospinning said solution, where the solution is:

  a) first dispersed at a collector rotation speed of more than 3,500 RPM, in order to produce a layer of aligned fibers,
  b) then the solution is dispersed at a collector rotation speed of less than 1,000 RPM, in order to produce a layer of randomly organized fibers, and;
  c) finally the solution is dispersed in the same condition than in step a).

Yet preferably, the method disclosed above comprises:

- preparing a solution of fibroin, wherein the solution comprises between 8% and 10% fibroin, and further comprises poly(ethylene oxide), and ;
- forming fibroin fibers by electrospinning said solution, where the solution is:

  d) first dispersed at a collector rotation speed of more than 3,500 RPM, in order to produce a layer of aligned fibers,
  e) then the solution is dispersed at a collector rotation speed of less than 500 RPM, in order to produce a layer of randomly organized fibers, and;
  f) finally the solution is dispersed in the same condition than in step a).

In an advantageous embodiment, the method disclosed above comprises:

- preparing a solution of fibroin, wherein the solution comprises 8% fibroin, and further comprises poly(ethylene oxide), and ;
- forming fibroin fibers by electrospinning said solution, where the solution is:

> g) first dispersed at a collector rotation speed of 4000 RPM, in order to produce a layer of aligned fibers,
> h) then the solution is dispersed at a collector rotation speed of 400 RPM, in order to produce a layer of randomly organized fibers, and;
> i) finally the solution is dispersed in the same condition than in step a).

Poly(ethylene oxide) is classically used with biological fibers in electrospinning methods known in the art. The person skilled in the art may thus adjust its concentration in the solution comprising fibroin according to his own knowledge. Typically, the fibroin solution of the invention comprises about 1% poly(ethylene oxide).

In a third embodiment, the invention pertains to a polymer scaffold comprising the fibrous polymer material of the invention.

The polymer scaffold of the invention can be formed into a variety of shapes. The fibrous polymer material of the invention has been found particularly useful when used in nerve guidance conduits, which typically have a tubular shape. In an advantageous embodiment, the polymer scaffold of the invention has a tubular shape.

The preparation of a polymer scaffold using the fibrous polymer material of the invention can easily be performed using methods known in the art. Typically, the fibrous polymer material is rolled around a cylindrical shape, in order to obtain the shape of a conduit. To insure maintenance of the shape over time, the fibrous polymer material may be treated so as to induce the formation of β-sheets within the fibroin fibers.

Most tubular polymer scaffolds used as nerve guidance conduits typically have a hollow inner part. However, the inventors have designed complex tubular shapes, wherein inner part is not hollow but structured into multiple inner conduits, which have a favorable impact on tissue recovery, in particular when used as a nerve guidance conduit. Without being bound by theory, the multi-channeled design increases the surface area compared to hollow nerve guides, and it is assumed that the additional surface area further aids in axon guidance.

In an advantageous embodiment, the polymer scaffold of the invention has a tubular shape which inner part has a multi-channeled structure.

Preferably, when the polymer scaffold of the invention has a tubular shape, the fibroin fibers of the first layer of the fibrous polymer material of the invention are aligned with the axis of the tube. In other terms, in the polymer scaffold of the invention, the fibroin fibers of the first layer of the fibrous polymer material of the invention are parallel to the axis of said tube.

For ease of preparation, the polymer scaffold of the invention may be manufactured using the fibrous polymer material of the invention in the form of sheets. A single sheet of the fibrous polymer material of the invention may therefore be used to prepare a polymer scaffold having a tubular shape, in which case the wall of the tube will of course have the same thickness as the thickness of the one sheet of fibrous polymer material it is made of.

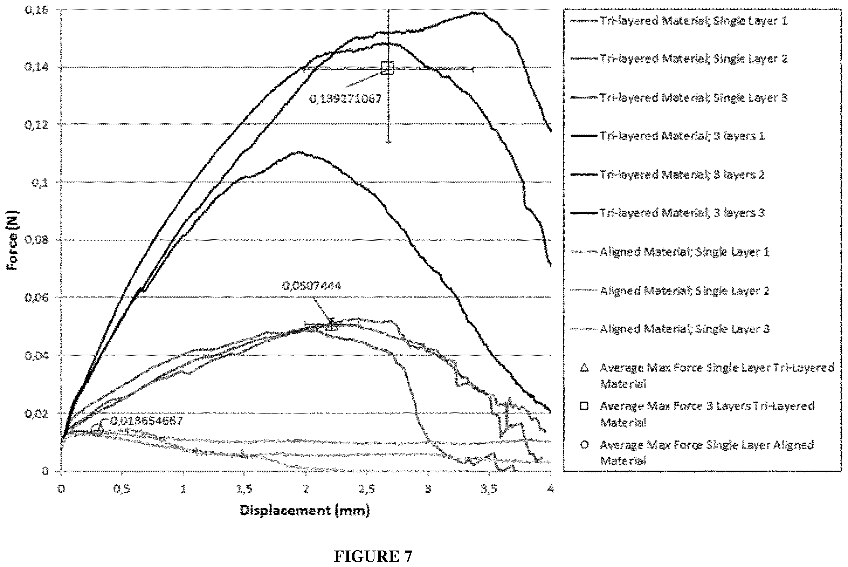

Although one sheet of the fibrous polymer material of the invention may prove sufficient, it may be advantageous to combine two or more of said sheets, put on top of each other, so as to increase the tensile strength of the overall material used when manufacturing the polymer scaffold. This increase in tensile strength and tear strength has for instance been demonstrated by the inventors by combining three sheets of the fibrous polymer material of the invention, as shown in figure 7 .For instance, two or more sheets of the fibrous polymer material of the invention may be combined and rolled so as to obtain a polymer scaffold having a tubular shape, in which case the wall of the tube will of course have a thickness corresponding to the some of the thickness of the sheets of fibrous polymer material it is made of.

In an advantageous embodiment, the polymer scaffold of the invention comprises 2or more, preferably 3, sheets of the fibrous polymer material of the invention, wherein said sheets are combined.

In an embodiment, the polymer scaffold of the invention has a tubular shape, wherein the inner part has a multi-channeled structure, and an external part formed of two or more sheets of the fibrous polymer material of the invention, wherein said sheets are combined. Preferably, the external part formed of two or more sheets of the fibrous polymer material of the invention extends on each end of the tube beyond the multi-channeled structure. This feature, by forming hollow pockets on each end of the tube, has been shown by the inventors to facilitate suturing of the polymer scaffold to the nerves, when used as a nerve guidance conduit.

The inventors have moreover determined a particularly useful embodiment of the polymer scaffold of the invention, wherein the features are optimal when said scaffold is to be used as a nerve guidance conduit (NGC).

In an advantageous embodiment, the polymer scaffold of the invention has a tubular shape, wherein the inner part has a multi-channeled structure, and an external part formed of two or more sheets of the fibrous polymer material of the invention, wherein said sheets are combined, and wherein said external part formed of two or more sheets of the fibrous polymer material of the invention extends on each end of the tube beyond the multi-channeled structure. Preferably, in this embodiment, the fibrous polymer material of the invention is functionalized as disclosed above.

Another aspect of the invention is a method to produce a polymer scaffold, comprising folding the fibrous polymer material of the invention into a shape of interest, and treating said fibrous polymer material so as to induce β-sheet formation, preferably with a methanol solution, yet preferably a solution comprising at least 99% methanol, or water vapor annealing.

Methods for inducing β-sheet formation of fibroin using methanol aqueous solution are known in the art, and for instance have been thoroughly described in Tsukada et al. (Journal of Polymer Science Part B Polymer Physics; 32(5):961-968; 1994). Methods for inducing β-sheet formation of fibroin using water vapor annealing are known in the art, and for instance have been thoroughly described in Xu et al. (Biomacromolecules;12(5):1686-96, 2011).

Advantageously, in the method to produce a polymer scaffold, the fibrous polymer material of the invention is folded into a tubular shape, preferably into a tubular shape which inner part has a multi-channeled structure.

Preferably, the step of folding the fibrous polymer material of the invention into a shape of interest is performed using a Teflon-coated object having the shape of interest, yet preferably a Teflon-coated cylinder.

Preferably, the step of treating the fibrous polymer material so as to induce β-sheet formation with methanol solution is performed for between 1 to 15 minutes, preferably for 3 to 8 minutes, preferably for 5 minutes.

Preferably, the step of treating the fibrous polymer material so as to induce β-sheet formation using water vapor annealing is performed for between 3 to 8 hours, preferably for 3 to 6 hours, preferably for 4 hours.

Advantageously, the step of treating the fibrous polymer material with methanol if followed by a step of drying the fibrous polymer material, preferably air drying the fibrous polymer material. The step of drying the fibrous polymer material is preferably performed for between 30 to 90 minutes, preferably for about 60 minutes.

Preferably, in particular when the polymer scaffold is to be used an implant, the method may further comprise a step of extracting poly(ethylene oxide) from the fibrous polymer material, preferably by rinsing the polymer scaffold in a buffer solution, such as a PBS buffer solution. Advantageously, the step of extracting poly(ethylene oxide) from the fibrous polymer material is performed by immersing said fibrous polymer material overnight at 37°C in Milli-Q water, then rinsing said fibrous polymer material 3 times in Milli-Q water at room temperature.

**FIGURE LEGEND**

Figure 1: Typical electrospinning set up; a) syringe with polymer solution; b) syringe pump; c) spinneret; d) Taylor cone; e) rotating drum collector; f) high voltage power supply.

Figure 2: SEM images of A) beginning of third aligned layer on top of middle random layer; B) final top aligned layer; C&D) cross section of tri-layered material after snap frozen in liquid nitrogen and cut longitudinally.

Figure 3: Average maximum stress measurements (left) and average Young's Modulus measurements (right) of silk materials and a native sciatic nerve.

Figure 4: SEM images of first layer of aligned fibers.

Figure 5: Cross section of a tubular polymer scaffold according to the invention, which inner part has a multi-channeled structure.

Figure 6. Implantation process. (A) the rat sciatic nerve was exposed and secured; (B) the implant was sutured twice at 180° at the distal nerve segment; (C) the proximal nerve segment was placed inside implant cavity; (D) the implant was sutured twice at 180° at the proximal nerve segment

Figure 7: Tear strength analysis of material formed of either a sheet comprising a single layer of aligned fibers, a sheet of the fibrous polymer material of the invention (comprising three layers of fibers), and a combination of three sheets of the fibrous polymer material of the invention.

Figure 8: Average force-displacement at maximum tensile force of material formed of either a sheet comprising a single layer of aligned fibers, a sheet of the fibrous polymer material of the invention (comprising three layers of fibers), and a combination of three sheets of the fibrous polymer material of the invention.

Figure 9: cross section of the rat sciatic nerve distal to the implantation site 4 months after surgery using the presented silk fibroin device (not functionalized with any biomolecules). Immunostaining includes DAPI (stains the cell nuclei), β-III Tubulin (stains axons of neurons), and Phalloidin (stains actin, extracellular matrices).

Figure 10: cross section of a healthy rat sciatic nerve. Immunostaining includes DAPI (stains the cell nuclei), β-III Tubulin (stains axons of neurons), and Phalloidin (stains actin, extracellular matrices).

**EXAMPLES**

**Methods**

1. Preparation of Silk Fibroin Solution

Silk cocoons from the *Bombyx mori* silkworm were cut into small pieces and boiled for 30 minutes in a 0.02 M Na2CO3 (Sigma-Aldrich) aqueous solution. The silk fibroin (SF) fibers were rinsed three times in cold DI water then allowed to dry for 24 hours at room temperature. The dry SF fibers were dissolved in a 9.3 M solution of LiBr (Sigma-Aldrich) for up to 4 hours at 60°C.

The SF solution was dialyzed against DI water using Slide-a-Lyzer dialysis cassettes (Thermo Fisher Scientific) for 3 days to remove salts. The SF solution was centrifuged twice to remove solid particles. The final concentration was about is between 6 and 8 wt%. A 10 wt% SF solution was achieved by allowing water to evaporate from the solution overnight. The resulting 10 wt% SF solution was stored at 4°C for up to 6 weeks.

2. Electrospinning

A 10 wt% SF solution was mixed at a ratio of 4:1 with a 5 wt% solution of poly(ethylene oxide) (PEO, average Mv ~900,000, Sigma-Aldrich) resulting in a 8 wt% SF (1 wt% PEO) spinning solution. The spinning solution was dispensed through a 19 G stainless steel spinneret (Ramé-Hart Instrument Co.), at a flow rate of 1 mL/hr while a voltage between 10-15 kV is administered to the needle. The electrospinning rotating drum collector made in-house had a diameter of 12.8 cm, a thickness of 3 cm, and was positioned on the same axis as the spinneret at a distance of 12.5 cm from the tip of the spinneret. The entire system was enclosed in a plexiglass containment area with a controlled humidity range of 25-35%. To obtain an aligned fiber material (SF-A), the spinning solution was dispensed continuously for 90 minutes while the collector rotated at 4,000 RPM. To obtain a tri-layered (aligned--random-aligned) fiber material (SF-ARA), the spinning solution was dispensed for a total of 90 minutes: 30 minutes with a collector rotation speed at 4,000 RPM, followed immediately by 30 minutes at 400 RPM, then 30 minutes at 4,000 RPM. To obtain a random fiber material (SF-R), the spinning solution was dispensed continuously for 90 minutes with a collector rotation speed at 400 RPM. Standard aluminum foil was used to cover the collector surface before each process to allow for easier sample recovery.

3. Scanning Electron Microscope Imaging

Electrospun fiber materials were gold sputter coated and analyzed using a scanning electron microscope (SEM) at the surface and the edge of the material. To observe the three layers of the SF-ARA samples, the material was snap frozen in liquid nitrogen before being cut for a clean, blunt edge for analyses.

4. Tensile Strength Testing

Electrospun SF-R, SF-A, and SF-ARA samples were rolled (perpendicular to fiber alignment for samples SF-A and SF-ARA) 4 rotations to produce a hollow tube. The tubes were water vapor annealed for 4 hours then immersed in Milli-Q water overnight at 37°C to extract the PEO from the fibers. The tubes were subsequently rinsed then immersed in PBS for storage.

To perform tensile strength tests, each sample with a gauge length of 3 mm was consecutively secured lengthwise between the upper and lower holding grips. Each trial was carried out at a cross--head speed of 0.06 mm/s while recording load measurements in N every 100 ms. Assays for each material were done in triplicate.

5. Implant Fabrication -Layered, multi-channel Design

The tri-layered electrospun material was slowly peeled from the aluminum foil and a 5 mm (parallel to aligned fibers) by 3 cm (perpendicular to aligned fibers) rectangle of the material was cut using a clean scalpel blade. The long side of the material was then folded in half in order to enclose the material surface previously in contact with the aluminum foil obtaining a 5 mm by 1.5 cm rectangle. The material was then rolled while adding a Teflon-coated stick (0.2 mm diameter) every half-rotation. Once completely rolled, the free edge of the material was tightly pressed to the outside of the rolled tube and the tube was immediately immersed in methanol for 5 min to induce β-sheet formation. The tube was allowed to air-dry for 1 hr and the Teflon-coated sticks were then removed resulting in a 5 mm long multi-channel tube.

From the tri-layered electrospun material, a 7 mm (parallel to aligned fibers) by 3 cm (perpendicular to aligned fibers) rectangle was cut and placed so that the surface previously in contact with the aluminum foil is face down. The multi-channel tube was placed at the bottom center on this rectangle (aligned fibers in the same direction). The larger material was then rolled around the tube for 3 rotations to create a "jacket". The edge was tightly pressed to the tube and the entire system was water vapor annealed at room temperature for 4 hours to induce β-sheet formation. The implants were immersed in Milli-Q water overnight at 37°C then rinsed three times in order to eliminate traces of PEO. The implants were then sterilized in 70% ethanol overnight. They were finally rinsed three times with sterile water and immersed in sterile PBS for storage.

6. Surgery

Male Sprague Dawley rats of 12 six week old were each anesthetized with vetflurane throughout the entire operation. The rats' left hind limbs were each shaved and sterilized. For each rat, an incision parallel to the femur was made and the sciatic nerve was exposed, isolated and fixed with two micro clips 10 mm apart. The nerve was severed at the distal end of the fixed section and the implant's outer layer was sutured twice to the distal segment's epineurium at 180° enveloping the epineurium and nerves fascicles. From the proximal nerve segment, a 3-4 mm portion of nerve was extracted and discarded. The exposed perineurium and fascicles of the proximal nerve segment was inserted into the opening of the implant, and the implant's outer layer was sutured twice to the epineurium at 180°. The operated area was then cleaned and the wound was closed and sutured.

**Results**

Electro spinning

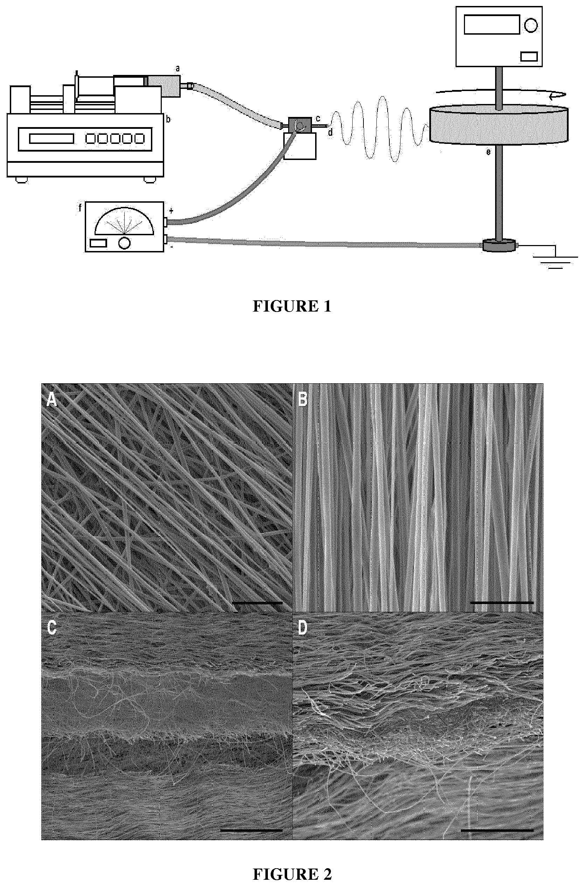

After a total of 90 minutes of electro spinning, a fibrous silk fibroin material 3 cm wide, 40 cm long, and 0.08 mm ($\pm$0.01 mm) thick was obtained. Fiber diameters were analyzed using ImageJ software and varied between 250 nm and 650 nm with an average of 390 nm. The SF-ARA material clearly presented three distinct layers visible through SEM analyses (Figure 2). The aligned-fiber surface of the SF-ARA was also shown to completely cover the random fiber layer in the center (figure 2B) verifying that the guidance factor of this material was not compromised.

The electrospinning setup is shown in Figure 1

Tensile Strength Testing

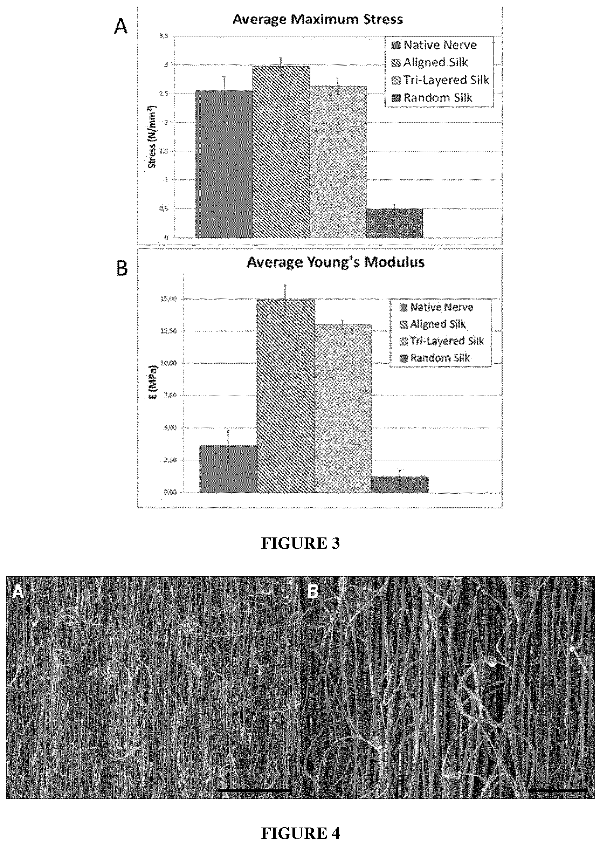

Average tensile strength results of material samples SF-A, SF-ARA, and SF-R are represented in the stress---strain graph displayed in figure 3. Stress and strain were calculated using equations (1) and (2) respectively:

$$(1) \quad \sigma = \frac{F}{A}$$

$$(2) \quad \varepsilon = \frac{\Delta l}{l_0}$$

Where $\sigma$ is tensile stress, F is force, A is cross sectional area (calculated by material thickness multiplied by the width of material: A=0.08mm$\times$30mm), $\varepsilon$ is strain, $\Delta l$ is the change in length, and $l_0$ is initial length.

The SF-A and SF-ARA samples were shown to possess similar average maximum stress measurements of 2.98 N/mm$^2$ and 2.63 N/mm$^2$ respectively which were slightly higher than that of a native sciatic nerve (2.55 N/mm$^2$) and significantly higher than that of SF-R demonstrating an average maximum stress of 0.49

N/mm$^2$. However, the SF-A samples were found to be significantly less resistant to elongation than both the SF-ARA and SF-R samples exhibiting an average strain of 0.85 mm/mm at the average maximum stress occurring immediately before rupture. This behavior is comparable to the native nerve which had a strain value of 0.80 mm/mm. SF-ARA and SF-R samples had similar strain measurements at their respective average maximum stress points (2.63 mm/mm and 2.50 mm/mm respectively). SF-ARA and SF-R samples also showed some elasticity before permanent deformation whereas the average SF-A curve does not present any apparent elastic characteristics. Finally, in contrast to the SF-A samples, SF-ARA samples presented a gradual decline in stress measurements following the rupture point (immediately after the maximum average stress point); SF-A samples experienced an abrupt/sharp decline in stress corresponding to a more violent rupture. This result suggests that, contrary to SF-A samples, SF-ARA samples preserve some continuity after initial rupture.

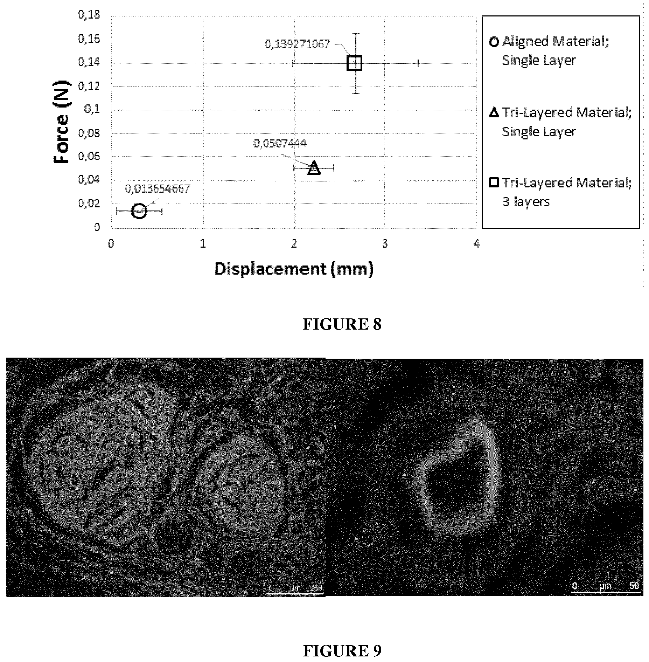

Average tensile strength results of sutured SF-ARA and SF-A material samples are represented in figures 7 and 8. A single layer or 3 layers of the SF-A or SF-ARA material were sutured (without a knot) at 1mm from the longitudinal edge of each sample. The non-sutured edge and the two suture ends were secured within the clamps of the apparatus. While the average sutured single layer of the SF-ARA material resisted more than 3.5 times the tensile force of the average sutured single layer of the SF-A material before tearing, the average sutured 3-layered SF-ARA material resisted more than 2.5 times the tensile force of the sutured single layer of the SF-ARA material before tearing. The sutured single and 3-layer SF-ARA material samples, despite reaching their maximum force, conserved substantial continuity until the maximum displacement reached during the experiment (4mm). The sutured single layer of the SF-A material lost continuity or virtually all continuity before the maximum displacement reached during the experiment (4mm). The 3-layered SF-ARA material therefore presents a superior resistance to tearing than the sutured single layer samples.

Implant Design/Preparation

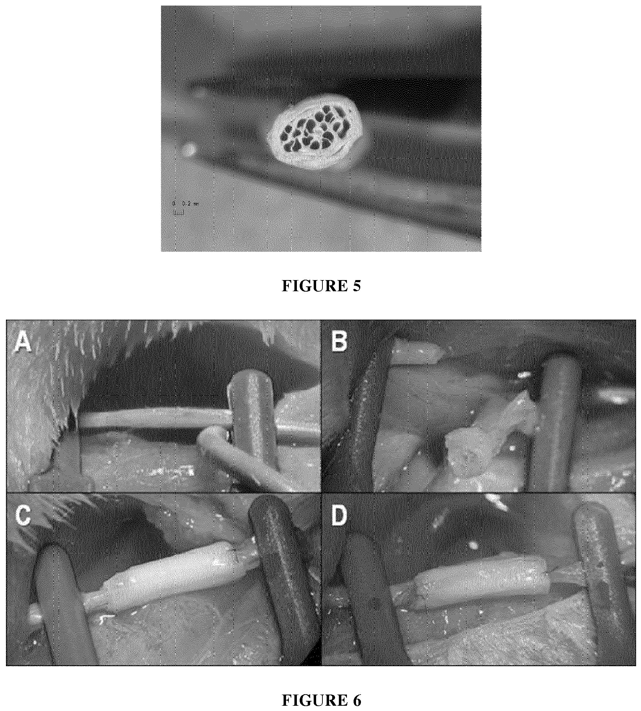

After SEM analyses of the tri-layered material, it was discovered that the act of peeling the material from the aluminum foil collector caused many of the aligned fibers to stretch, break, and coil due to the slight adhesion of the fibers to the foil (Figure 4). This is an undesirable consequence since the coiling of the fibers negates/nullifies the alignment of the fibers and therefore eliminates the surface guidance factor. Therefore the material was folded in half in order to conceal the flawed surface before rolling the material into a multi-channel tube (Figure 5). The implants had a final diameter of approximately 1.1 mm.

Surgery

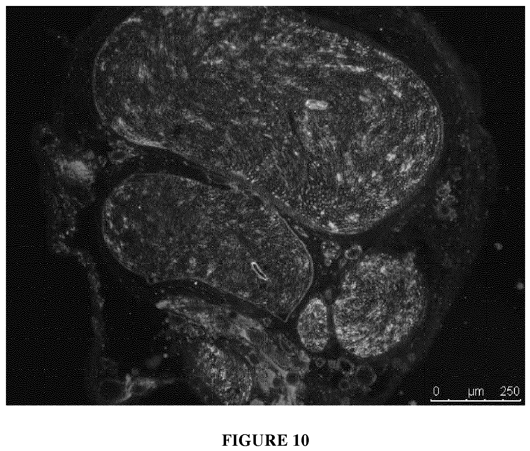

Surgery was successfully carried out on the right sciatic nerve of all 12 rats by following a 4-step process depicted in Figure 6. The implant was easily sutured to the distal nerve segment while avoiding any disturbance to the nerve fascicles due to the small hollow pocket provided by the "jacket" enveloping the multichannel conduit. The implant was then readily positioned for the sutures to the proximal nerve segment as the hollow pocket secured the nerve in the correct position for the final sutures (demonstrated in Figure 6). The tri-layered material was found to have sufficient tear strength to withstand suture punctures which was not the case with the SF-A material. The results of the implantation was assessed 4 and 8 months after surgery. The rat sciatic nerve was fixed and cross-section samples were stained using DAPI, anti-$\beta$III tubulin antibodies and Phalloidin (Figure 9). A healthy rat sciatic nerve was treated similarly, and used as control (Figure 10).

The observation confirms that there was a high level of nerve regeneration at 8 months after the implantation of the silk guidance conduit. As there exists several types of neurons within the sciatic nerve, and they all vary in axon diameter, 50 of the largest axons in a $37500\mu m^2$ section area were measured in the 8 month sample as well as in the control sample. The average diameter of axons found in the 8 month cross section was found to be $1.34\mu m \pm 0.24\mu m$ while the average diameter of axons found in the control cross section was found to be $1.48\mu m \pm 0.24\mu m$. This therefore demonstrates a clear improvement of nerve regeneration between after 8 months supported by a silk guidance conduit that approaches the results obtained from healthy nerve control samples.

## Claims

1. A fibrous polymer material, comprising:

   - a first layer of aligned fibroin fiber ;
   - a second layer of fibroin fibers, wherein the fibroin fibers are randomly oriented within said second layer ;
   - a third layer of aligned fibroin fibers.

2. The fibrous polymer material of claim 1, wherein the fibroin is from silkworms, preferably silkworms chosen form a species chosen in the list consisting of *Bombyx mori, Anthéroea Yama-Maî* and *Anthéroea Pernyï,* yet preferably from *Bombyx mori.*

3. The fibrous polymer material of claim 1 or 2, wherein the fibroin fibers have a diameter of between 100 nm and 900 nm, preferably of between 250 nm and 650 nm.

4. The fibrous polymer material of any one of claims 1 to 3, wherein it is functionalized by the addition of at least one biological molecule, preferably chosen from the list consisting of neuronal growth factor (NGF), ciliary neurotrophic factor (CNTF) and epidermal growth factor (EGF), neurotrophin 3 (NT-3), brain derived neurotrophic factor (BDNF) and Neurotrophin 4/5 (NT-4/5).

5. A method for producing a fibrous polymer material according to any one of claims 1 to 4, comprising preparing a solution of fibroin and poly(ethylene oxide), and forming fibroin fibers by electrospinning said solution.

6. The method according to claim 5, wherein it comprises:

   - preparing a solution of fibroin, wherein the solution comprises between 4% and 15% fibroin, and further comprises poly(ethylene oxide), and ;
   - forming fibroin fibers by electrospinning said solution, where the solution is:

     a) first dispersed at a collector rotation speed of more than 2,000 RPM, in order to produce a layer of aligned fibers,
     b) then the solution is dispersed at a collector rotation speed of less than 1,000 RPM, in order to produce a layer of randomly organized fibers, and;
     c) finally the solution is dispersed in the same condition than in step a).

7. A polymer scaffold, comprising the fibrous polymer material of claim 1.

8. The polymer scaffold of claim 7, wherein it has a tubular shape.

9. The polymer scaffold of claim 7 or 8, wherein the fibroin fibers of the first layer of the fibrous polymer material of the invention are aligned with the axis of the tube.

10. The polymer scaffold of any one of claims 7 to 9, wherein it has a tubular shape which inner part has a multi-channeled structure.

11. The polymer scaffold of any one of claims 7 to 10, wherein it comprises 2 or more, preferably 3, sheets of the fibrous polymer material of the invention, wherein said sheets are combined.

12. The polymer scaffold of any one of claims 7 to 11, wherein it has a tubular shape, wherein the inner part has a multi-channeled structure, and an external part formed of two or more sheets of the fibrous polymer material of the invention, wherein said sheets are combined.

13. The polymer scaffold of any one of claims 7 to 12, wherein it has a tubular shape, wherein the inner part has a multi-channeled structure, and an external part formed of two or more sheets of the fibrous polymer material of the invention, wherein said sheets are combined, and wherein said external part formed of two or more sheets of the fibrous polymer material of the invention extends on each end of the tube beyond the multi-channeled structure.

14. The polymer scaffold of claim 13, wherein the fibrous polymer material is functionalized by the addition of at least one biological molecule, preferably chosen from the list consisting of neuronal growth factor (NGF), ciliary neurotrophic factor (CNTF) and epidermal growth factor (EGF), neurotrophin 3 (NT-3), brain derived neurotrophic factor (BDNF) and Neurotrophin 4/5 (NT-4/5).

15. A method to produce a polymer scaffold of any one of claims 7 to 14, comprising folding the fibrous polymer material of any one of claims 1 to 5 into a shape of interest, and treating said fibrous polymer material so as to induce β-sheet formation, preferably with methanol aqueous solution or water vapor annealing.

**FIGURE 1**

**FIGURE 2**

**FIGURE 3**

**FIGURE 4**

**FIGURE 5**

**FIGURE 6**

FIGURE 7

EP 3 427 764 A1

**FIGURE 8**

**FIGURE 9**

**FIGURE 10**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MADDURI S ET AL: "Trophically and topographically functionalized silk fibroin nerve conduits for guided peripheral nerve regeneration", BIOMATERIALS, ELSEVIER SCIENCE PUBLISHERS BV., BARKING, GB, vol. 31, no. 8, 1 March 2010 (2010-03-01), pages 2323-2334, XP026870602, ISSN: 0142-9612 [retrieved on 2009-12-08] * Abstract, p. 2324, col. 1, par. 3 to col. 2, par 4; p. 2332, col. 2, par. 2 * ----- | 1-15 | INV. A61L27/22 A61L27/34 A61L27/50 A61L27/54 A61L27/56 |
| X | WO 02/49536 A2 (NICAST LTD [IL]; DUBSON ALEXANDER [IL]; BAR ELI [IL]) 27 June 2002 (2002-06-27) * p. 1, l. 5-8; p. 17, l. 26 to p. 18, l. 13; claim 1 * ----- | 1-15 | |

TECHNICAL FIELDS
SEARCHED     (IPC)

A61L

The present search report has been drawn up for all claims

| Place of search | Date of completion of the search | Examiner |
|---|---|---|
| The Hague | 11 January 2018 | Cadamuro, Sergio |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
 
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 17 30 5917

This annex lists the patent family members relating to the patent documents cited in the above-mentioned European search report.
The members are as contained in the European Patent Office EDP file on
The European Patent Office is in no way liable for these particulars which are merely given for the purpose of information.

11-01-2018

| Patent document cited in search report | | Publication date | Patent family member(s) | | Publication date |
|---|---|---|---|---|---|
| WO 0249536 | A2 | 27-06-2002 | AT | 460903 T | 15-04-2010 |
| | | | AT | 462371 T | 15-04-2010 |
| | | | AU | 1634002 A | 01-07-2002 |
| | | | AU | 1634202 A | 01-07-2002 |
| | | | AU | 2249402 A | 01-07-2002 |
| | | | AU | 2002222494 A8 | 19-02-2009 |
| | | | CA | 2432156 A1 | 27-06-2002 |
| | | | CA | 2432159 A1 | 27-06-2002 |
| | | | CA | 2432164 A1 | 27-06-2002 |
| | | | CN | 1489513 A | 14-04-2004 |
| | | | CN | 1599582 A | 23-03-2005 |
| | | | CN | 1635861 A | 06-07-2005 |
| | | | EP | 1353606 A2 | 22-10-2003 |
| | | | EP | 1355677 A2 | 29-10-2003 |
| | | | EP | 1578306 A2 | 28-09-2005 |
| | | | IL | 156513 A | 13-04-2008 |
| | | | IL | 156514 A | 18-11-2009 |
| | | | JP | 4145143 B2 | 03-09-2008 |
| | | | JP | 2004529673 A | 30-09-2004 |
| | | | JP | 2004532665 A | 28-10-2004 |
| | | | JP | 2005507464 A | 17-03-2005 |
| | | | MX | PA03005551 A | 24-10-2003 |
| | | | MX | PA03005552 A | 24-10-2003 |
| | | | MX | PA03005553 A | 23-08-2004 |
| | | | US | 2002084178 A1 | 04-07-2002 |
| | | | US | 2004053553 A1 | 18-03-2004 |
| | | | US | 2004054406 A1 | 18-03-2004 |
| | | | US | 2004096532 A1 | 20-05-2004 |
| | | | US | 2004096533 A1 | 20-05-2004 |
| | | | WO | 0249535 A2 | 27-06-2002 |
| | | | WO | 0249536 A2 | 27-06-2002 |
| | | | WO | 0249678 A2 | 27-06-2002 |

-------------------------------------------------------------------------

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KUNDU et al.** *Advanced Drug Delivery Reviews,* 2013, vol. 65 (4), 457-470
- **WITTMER et al.** *Acta Biomater,* 2011, vol. 7 (10), 3789-95
- **WITTMER et al.** *Adv Funct Mater.,* 2011, vol. 21 (22), 4202
- **DINIS et al.** *PLoS One.,* 2014, vol. 9 (10), e109770
- **DINIS et al.** *ACS Appl Mater Interfaces,* 2014, vol. 6 (19), 16817-26
- **TSUKADA et al.** *Journal of Polymer Science Part B Polymer Physics,* 1994, vol. 32 (5), 961-968
- **XU et al.** *Biomacromolecules,* 2011, vol. 12 (5), 1686-96